(19) Europäisches Patentamt
European Patent Office
Office européen des brevets

(11) **EP 4 613 709 A1**

(12) **EUROPEAN PATENT APPLICATION**
published in accordance with Art. 153(4) EPC

(43) Date of publication:
**10.09.2025 Bulletin 2025/37**

(21) Application number: 23885670.2

(22) Date of filing: 27.10.2023

(51) International Patent Classification (IPC):
*C01B 39/44* (2006.01)      *B01D 15/00* (2006.01)
*A61M 1/16* (2006.01)      *A61M 1/36* (2006.01)
*B01J 20/18* (2006.01)      *B01J 20/28* (2006.01)

(52) Cooperative Patent Classification (CPC):
**A61M 1/16; A61M 1/36; B01D 15/00; B01J 20/06;
B01J 20/10; B01J 20/18; B01J 20/28; C01B 39/44**

(86) International application number:
**PCT/JP2023/038862**

(87) International publication number:
**WO 2024/095914 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 02.11.2022  JP 2022176351
16.08.2023  JP 2023132534

(71) Applicant: **Tosoh Corporation**
**Yamaguchi 746-8501 (JP)**

(72) Inventors:
• **TSUCHIYA Kazuyoshi**
**Shunan-shi Yamaguchi 746-8501 (JP)**
• **OKANIWA Hiroshi**
**Shunan-shi Yamaguchi 746-8501 (JP)**
• **NAKAZAWA Naoto**
**Shunan-shi Yamaguchi 746-8501 (JP)**

(74) Representative: **TBK**
**Bavariaring 4-6**
**80336 München (DE)**

(54) **ZEOLITE MOLDED BODY**

(57)     An object is to provide at least any of zeolite-containing shaped bodies that can remove potassium from a dialysis effluent with less dissolution of aluminum compared to the conventional treatment of dialysis effluent using a zeolite, and a method of adsorbing potassium using the shaped bodies.

## Description

TECHNICAL FIELD

The present disclosure relates to zeolite shaped bodies.

BACKGROUND ART

Dialysis is the process of artificially removing wastes from blood using a dialysis fluid. Wastes, mainly potassium, in blood are removed by contact of the blood with a dialysis fluid through a semipermeable membrane. Dialysis consumes a large amount of dialysis fluid per session and is performed frequently. Thus, large amounts of dialysis fluid are discharged and treated as an effluent (a dialysis effluent) after dialysis. Home dialysis in which patients receive dialysis at home recently attracts attention from the point of view of improving the quality of life, but its widespread use is interfered with by the handling of dialysis effluent.

Incidentally, zeolites are known as potassium adsorbents that are easily available compared to other adsorbents, such as ion exchange resins. Thus, studies are underway on the use of zeolites in the treatment of dialysis effluent for the purpose of treating and regenerating the dialysis effluent.

For example, Patent Document 1 reports that a dialysis effluent is treated with FAU-type zeolites having different $SiO_2/Al_2O_3$ ratios to remove potassium from the dialysis effluent and to regenerate the dialysis effluent.

PRIOR ART DOCUMENTS

PATENT DOCUMENTS

Patent Document 1: U.S. Patent Application Publication No. 2015/0297815

DISCLOSURE OF INVENTION

TECHNICAL PROBLEM

However, treating a dialysis effluent with the zeolites described in Patent Document 1 encounters a problem in which aluminum is dissolved out during the potassium removal and is mixed into the dialysis effluent.

An object of the present disclosure is to provide at least any of zeolite-containing shaped bodies that can remove potassium from a dialysis effluent with less dissolution of aluminum compared to the conventional treatment of dialysis effluent using a zeolite, and a method of adsorbing potassium using the shaped bodies.

SOLUTION TO PROBLEM

The present inventors studied zeolites for use in the removal of potassium from a dialysis effluent focusing on the structure of zeolites and the form of zeolites when applied to the treatment of dialysis effluent. As a result, the present inventors have found that shaped bodies that comprise a zeolite with a specific structure and have controlled pores release less aluminum and exhibit outstanding potassium adsorption properties.

Specifically, the present invention is as described in the claims, and the gist of the present disclosure is as follows:

[1] Shaped bodies comprising a zeolite having an oxygen 8-membered ring structure, and at least any of a silica binder and a zirconia binder, the shaped bodies being such that the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies satisfy (1) or (2) below:

(1) the average aspect ratio is 1.55 or less, and
the particle size D50 is 200 $\mu$m or more and 600 $\mu$m or less;
(2) the average aspect ratio is greater than 1.55.

[2] The shaped bodies according to [1], wherein the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of greater than 1.55.

[3] The shaped bodies according to [1], wherein the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of 1.55 or less and a particle size D50 of 200 $\mu$m or more and 600 $\mu$m or less.

[4] The shaped bodies according to any one of [1] to [3], wherein the cumulative pore area of pores having a size of 5 nm or more and 300 nm or less is 10.0 $m^2$/g or more and 35.0 $m^2$/g or less.

[5] The shaped bodies according to any one of [1] to [4], wherein the cumulative pore volume of pores having a size of 5 nm or more and 300 nm or less is 0.35 mL/g or more and 0.70 mL/g or less.

[6] The shaped bodies according to any one of [1] to [5], wherein the pore size corresponding to 50 vol% cumulative pore volume of the cumulative pore volume of pores having a size of 5 nm or more and 300 nm or less is 75 nm or more and 160 nm or less.

[7] The shaped bodies according to any one of [1] to [6], wherein the ratio of the cumulative pore volume of pores having a size of 5 nm or more and 10 nm or less to the cumulative pore volume of pores having a size of 5 nm or more and 300 nm or less is 0.1 vol% or more and 5.0 vol% or less.

[8] The shaped bodies according to any one of [1] to [7], wherein the zeolite has one or more framework structures selected from the group of FER structures, HEU structures, YFI structures, MAZ structures, CHA structures, MER structures and MOR structures.

[9] The shaped bodies according to any one of [1] to [8], wherein the zeolite has a silica to alumina molar ratio (a $SiO_2/Al_2O_3$ ratio) of 3 or more and 100 or less.

[10] A potassium adsorption method comprising a step of contacting the shaped bodies described in any one of [1] to [9] and a solution containing potassium ions with each other.

[11] A column comprising the shaped bodies described in any one of [1] to [9].

[12] A potassium adsorption system comprising the column described in [11].

ADVANTAGEOUS EFFECTS OF INVENTION

The present disclosure can provide at least any of zeolite-containing shaped bodies that can remove potassium from a dialysis effluent with less dissolution of aluminum compared to the conventional treatment of dialysis effluent using a zeolite, and a method of adsorbing potassium using the shaped bodies.

DESCRIPTION OF EMBODIMENTS

Hereinbelow, the present disclosure will be described by presenting an exemplary embodiment. The terms used in the embodiment are defined as described below. The configurations and the parameters disclosed in the present specification may be combined appropriately. The upper and lower limits of the values disclosed in the present specification may be combined appropriately, and such combinations are also included in the scope of the present disclosure. The main terms used in the present disclosure will be described below.

"Aluminosilicate" is a composite oxide that has a structure composed of repeated networks of aluminum (Al) and silicon (Si) via oxygen (O). Since aluminum and silicon have different numbers of charges, aluminosilicates contain counter cations, such as $H^+$ and $Na^+$, to compensate for charges. Among aluminosilicates, those that have a crystalline X-ray diffraction (hereinafter, also written as "XRD") peak in a powder XRD pattern are "crystalline aluminosilicates", and those that do not have a crystalline XRD peak are "amorphous aluminosilicates".

"Zeolite" is a compound that has a regular structure in which framework atoms (hereinafter, also written as "T atoms") are regularly arranged via oxygen (O), and the T atoms are at least any of metal atoms and/or metalloid atoms. For example, the metal atoms may be one or more selected from the group consisting of aluminum (Al), titanium (Ti), iron (Fe), zinc (Zn), gallium (Ga) and tin (Sn), and are preferably at least any of aluminum and iron, and more preferably aluminum. For example, the metalloid atoms may be at least one selected from the group consisting of boron (B), silicon (Si), germanium (Ge), arsenic (As), antimony (Sb) and tellurium (Te), and are preferably silicon.

"Zeolite analog" is a compound that has a regular structure in which T atoms are regularly arranged via oxygen, and the T atoms include at least atoms other than metals and metalloids (hereinafter, such atoms are also written as "nonmetal atoms"). For example, the nonmetal atoms may be phosphorus (P). Examples of the zeolite analogs include composite phosphorus compounds that contain phosphorus (P) as T atoms, such as aluminophosphates (AlPO) and silicoaluminophosphates (SAPO).

The "regular structure" in the zeolites and the zeolite analogs (hereinafter, also written as the "zeolite structure") is a framework structure that is specified by a structure code established by the Structure Commission of the International Zeolite Association (hereinafter, the structure code established above is also written simply as the "structure code"). For example, an FER structure is a framework structure specified by the structure code "FER". A zeolite structure can be identified as an FER structure by comparison to the XRD pattern described in Zeolite Framework Type FER at the website of the IZA Structure Commission (http://www.iza-structure.org/databases/) (hereinafter, the XRD pattern is also written as the "reference pattern"). Regarding the zeolite structures, the terms "framework structure", "crystal structure" and "crystalline phase" are of the same meaning.

In the present embodiment, "-type zeolite", such as "FER-type zeolite", means that the zeolite has a zeolite structure of the corresponding structure code, and preferably means that the zeolite is a crystalline aluminosilicate having a zeolite structure of the corresponding structure code.

"Oxygen 8-membered ring structure" is a ring structure having eight T atoms, and is also written as a micropore.

"Zeolite shaped bodies" are a composition formed by giving a certain shape to a powdery zeolite. The composition is not limited to one consisting of the zeolite alone, and may include, for example, at least any of a binder and a shaping aid, in addition to the zeolite. In the present disclosure, the terms "shaped bodies" and "zeolite shaped bodies" are used interchangeably.

Shaped bodies according to the present embodiment will be described below.

The shaped bodies according to the present embodiment comprise a zeolite having an oxygen 8-membered ring structure, and at least any of a silica binder and a zirconia binder, have a 10-100 nm pore volume ratio of 25 vol% or more and 70 vol% or less, and satisfy (1) or (2) below:

> (1) the average aspect ratio is 1.55 or less, and
> the particle size D50 is 200 $\mu$m or more and 600 $\mu$m or less;
> (2) the average aspect ratio is greater than 1.55.

The shaped bodies of the present embodiment are shaped bodies mainly composed of a zeolite, namely, zeolite shaped bodies, and, in particular, comprise a zeolite having an oxygen 8-membered ring structure (hereinafter, also written as the "8-membered ring zeolite"), and at least any of a silica binder and a zirconia binder (hereinafter, also written collectively as the "inorganic binder").

The zeolite contained in the shaped bodies of the present embodiment has an oxygen 8-membered ring structure. The oxygen 8-membered ring structures allow for easy entry of potassium into the pores inside the shaped bodies of the present embodiment during the treatment of dialysis effluent, and do not let the potassium adsorbed to the zeolite desorb easily from the zeolite. The 8-membered ring zeolite may be a zeolite having one or more framework structures selected from the group of ABW structures, ACO structures, AEI structures, AEN structures, AFN structures, AFT structures, AFX structures, ANA structures, APC structures, APD structures, ATN structures, ATT structures, ATV structures, AWO structures, AWW structures, BCT structures, BIK structures, BRE structures, CAS structures, CDO structures, CHA structures, DDR structures, DFT structures, EAB structures, EDI structures, EPI structures, ERI structures, ESV structures, FER structures, GIS structures, GME structures, GOO structures, HEU structures, IHW structures, ITE structures, ITW structures, JBW structures, KFI structures, LEV structures, LTA structures, LTL structures, MAZ structures, MER structures, MON structures, MOR structures, MTF structures, NSI structures, OFF structures, OWE structures, PAU structures, PHI structures, RHO structures, RTE structures, RTH structures, RWR structures, SAS structures, SAT structures, SAV structures, SIV structures, SZR structures, THO structures, TSC structures, UEI structures, UFI structures, VNI structures, YFI structures, YUG structures and ZON structures. In view of the fact that the selectivity for potassium is enhanced by how oxygen atoms are arranged in the oxygen 8-membered ring structure, the 8-membered ring zeolite is preferably a zeolite having one or more framework structures selected from the group of FER structures, HEU structures, YFI structures, MAZ structures, CHA structures and MOR structures, more preferably a zeolite having one or more framework structures selected from the group of FER structures, HEU structures, YFI structures, MAZ structures, CHA structures and MOR structures, still more preferably a zeolite having one or more framework structures selected from the group of FER structures, HEU structures, YFI structures and MOR structures, and further preferably a zeolite having an FER structure, an HEU structure, a YFI structure or an MOR structure. The 8-membered ring zeolite may be particularly preferably a zeolite having an FER structure, an MER structure or an MOR structure, and may be further preferably a zeolite having an FER structure (an FER-type zeolite).

The silica to alumina molar ratio (the $SiO_2/Al_2O_3$ ratio) in the 8-membered ring zeolite is preferably 3 or more, 7 or more, 10 or more, 13 or more, or 15 or more, and is preferably 100 or less, 80 or less, 50 or less, or 35 or less. Such a $SiO_2/Al_2O_3$ ratio tends to lead to an improved performance in adsorbing potassium from a dialysis effluent. The above upper and lower limits of the $SiO_2/Al_2O_3$ ratio may be combined appropriately; for example, the molar ratio may be 3 or more and 100 or less, 7 or more and 80 or less, or 15 or more and 35 or less.

To ensure that the performance in adsorbing potassium from a dialysis effluent will be improved more reliably, the cationic type of the 8-membered ring zeolite is preferably at least any of cationic type: Na (sodium) and cationic type: H (proton), and is more preferably cationic type: Na.

The primary particle size of the 8-membered ring zeolite is preferably 0.1 $\mu$m or more and 15 $\mu$m or less.

The particle size D50 of the 8-membered ring zeolite is preferably 1 $\mu$m or more and 100 $\mu$m or less.

The shaped bodies of the present embodiment comprise an inorganic binder, and preferably comprise a silica binder. The incorporation of a silica binder as an inorganic binder tends to result in less leaching of aluminum when the shaped bodies come into contact with a solution, such as a dialysis effluent. Furthermore, the strength of the shaped bodies tends to be enhanced as a result of the silica in the silica binder adhering to the 8-membered ring zeolite. On the other hand, the shaped bodies of the present embodiment may comprise a zirconia binder as an inorganic binder. The strength of the shaped bodies tends to be enhanced as a result of the zirconia in the zirconia binder adhering to the zeolite.

The silica binder is a compound mainly containing silicon (Si) and oxygen (O), preferably a compound including silica ($SiO_2$), and more preferably silica.

The zirconia binder is a compound mainly containing zirconium (Zr) and oxygen (O), preferably a compound including zirconia ($ZrO_2$), and more preferably zirconia.

The inorganic binder contained in the shaped bodies of the present embodiment may be either a silica binder or a zirconia binder or may include both.

The primary particle size of the inorganic binder is preferably 10 nm (0.010 $\mu$m) or more and 150 nm (0.150 $\mu$m) or less. To enhance the adhesion with the zeolite, the primary particle size of the inorganic binder is more preferably 10 nm (0.010 $\mu$m) or more and 20 nm (0.020 $\mu$m) or less. On the other hand, the primary particle size of the inorganic binder is more preferably 70 nm (0.070 $\mu$m) or more and 120 nm (0.120 $\mu$m) or less in order to prevent dissolution into a dialysis effluent.

The particle size D50 of the silica binder is preferably 15 $\mu$m or more and 40 $\mu$m or less. The particle size D50 of the zirconia binder is preferably 0.05 $\mu$m or more and 40 $\mu$m or less.

The particle size of the 8-membered ring zeolite and the particle size D50 of the inorganic binder are each the particle size corresponding to D50 (median size) in the volumetric grain size distribution obtained by the laser diffraction/scattering method using a general particle size distribution analyzer (for example, device name: MT-310011, manufactured by MicrotracBEL Corporation).

The shaped bodies of the present embodiment easily attain enhanced potassium adsorption properties while maintaining the strength of the shaped bodies. To ensure this, it is preferable that the mass of the inorganic binder per 100 parts by mass (anhydrous equivalent mass) of the 8-membered ring zeolite be 10 parts by mass or more, 15 parts by mass or more, or 20 parts by mass or more, and be 40 parts by mass or less, 30 parts by mass or less, or 25 parts by mass or less. For example, less aluminum leaching tends to be obtained when the mass of the inorganic binder per 100 parts by mass (anhydrous equivalent mass) of the 8-membered ring zeolite is 10 parts by mass or more and 40 parts by mass or less, 15 parts by mass or more and 30 parts by mass or less, or 20 parts by mass or more and 25 parts by mass or less.

The anhydrous equivalent mass of the 8-membered ring zeolite and that of the inorganic binder are each the mass after heat treatment at 600°C for 1 hour in an air atmosphere. The anhydrous equivalent mass of a shaping aid is the mass after heat treatment at 80°C for 1 hour in an air atmosphere.

The shaped bodies of the present embodiment are preferably such that the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of greater than 1.55. By satisfying this average aspect ratio, the shaped bodies exhibit high potassium adsorption performance regardless of the particle size D50.

**For the reason** that the increase in external surface area of the shaped bodies provides an increased area of contact with a dialysis fluid, the average aspect ratio is preferably greater than 1.55, more preferably greater than 1.55 and 2.70 or less, still more preferably 1.60 or more and 2.60 or less, and further preferably 1.80 or more and 2.55 or less.

In the present embodiment, the "aspect ratio" of the shaped body is a value expressed by the ratio of the long axis to the short axis of the shaped body, and the "average aspect ratio" is the average of such aspect ratios. The long axes and the short axes of the shaped bodies may be determined in such a manner that independent shaped bodies observed on a scanning electron microscope (hereinafter, also written as "SEM") are projected onto the same plane, and a longer side and a shorter side of each rectangle circumscribing the shaped body image are defined as the long axis [$\mu$m] and the short axis [$\mu$m], respectively. That is, the "aspect ratio" is always 1.00 or more. The SEM observation may be performed using a general scanning electron microscope (for example, device name: JSM-IT200, manufactured by JEOL Ltd.) under the following conditions:

Acceleration voltage: 6 kV
Magnification: $\times 30 + 10$

The average aspect ratio of the shaped bodies of the present embodiment may be calculated as the arithmetic mean of the aspect ratios of 50 $\pm$ 5 shaped bodies observed in the above manner.

The shaped bodies of the present embodiment preferably have an average short axis of 150 $\mu$m or more and 700 $\mu$m or less, more preferably 200 $\mu$m or more and 650 $\mu$m or less, and still more preferably 400 $\mu$m or more and 650 $\mu$m or

less. The "average short axis" of the shaped bodies is the average of the short axes of the shaped bodies, and may be calculated as the arithmetic mean of the short axes of 50 ± 5 shaped bodies observed by SEM in the same manner as described above.

The shaped bodies of the present embodiment may be such that the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of 1.55 or less and a particle size D50 of 200 $\mu$m or more and 600 $\mu$m or less.

When the average aspect ratio is 1.55 or less, the particle size D50 makes a greater contribution to the potassium adsorption performance. By combining the above average aspect ratio and particle size D50, the potassium adsorption performance tends to be improved. For the reason that the packing density of the shaped bodies is easily increased, the average aspect ratio is preferably greater than 1.00 and 1.55 or less, and more preferably 1.30 or more and 1.50 or less.

When the average aspect ratio of the shaped bodies of the present embodiment is 1.55 or less, the particle size D50 is 200 $\mu$m or more and 600 $\mu$m or less. If the particle size D50 of the shaped bodies is less than 200 $\mu$m, the flow rate of a dialysis effluent or the like will be slow. If, on the other hand, the particle size D50 is more than 600 $\mu$m, the shaped bodies themselves are too large. As a result, the shaped bodies will not allow a dialysis effluent to penetrate easily into the inside thereof and will exhibit a very low efficiency in removing potassium from the dialysis effluent. The particle size D50 of the shaped bodies of the present embodiment is preferably 250 $\mu$m or more, 300 $\mu$m or more, or 350 $\mu$m or more, and is preferably 550 $\mu$m or less, 500 $\mu$m or less, or 450 $\mu$m or less. The above upper and lower limits of the particle size D50 may be combined appropriately. For example, the amount of aluminum leaching tends to be reduced without causing a decrease in potassium adsorption properties when the particle size D50 is 250 $\mu$m or more and 550 $\mu$m or less, 300 $\mu$m or more and 500 $\mu$m or less, or 350 $\mu$m or more and 450 $\mu$m or less.

The particle size D50 of the shaped bodies is the particle size corresponding to D50 (median size) in the volumetric grain size distribution obtained by the laser diffraction/scattering method using a general particle size distribution analyzer (for example, device name: MT-310011, manufactured by MicrotracBEL Corporation). Specifically, the measurement conditions may be as follows:

Pretreatment: Drying in air atmosphere at 110°C for 1 hour
Measurement range: 0.7 to 1000 $\mu$m
Transmissivity: Transmissive
Shape: Aspheric
Refractive index: 1.66
Atmosphere: Air (dry)
Solvent refractive index: 1

In the shaped bodies of the present embodiment, the ratio of the cumulative pore volume of pores having a size of 10 nm or more and 100 nm or less (hereinafter, the ratio is also written as the "10-100 nm pore volume ratio") to the cumulative pore volume of pores having a size of 5 nm or more and 300 nm or less (hereinafter, also written as the "total pore volume") is 25 vol% or more and 70 vol% or less. If the 10-100 nm pore volume ratio of the shaped bodies is less than 25 vol%, the potassium adsorption capacity tends to be lowered. If the 10-100 nm pore volume ratio is more than 70 vol%, the shaped bodies tend to adsorb other ions and the potassium selectivity will be lowered. The 10-100 nm pore volume ratio of the shaped bodies of the present embodiment is preferably 30 vol% or more, 33 vol% or more, 43 vol% or more, or 53 vol% or more, and is preferably 69 vol% or less, 67 vol% or less, or 65 vol% or less. The above upper and lower limits of the 10-100 nm pore volume ratio may be combined appropriately; for example, the 10-100 nm pore volume ratio may be 25 vol% or more and 70 vol% or less, 30 vol% or more and 70 vol% or less, 33 vol% or more and 69 vol% or less, 43 vol% or more and 67 vol% or less, or 53 vol% or more and 65 vol% or less.

In the shaped bodies of the present embodiment, the cumulative pore area of pores having a size of 5 nm or more and 300 nm or less (hereinafter, the area is also written as the "total pore area") is preferably 10.0 m$^2$/g or more, 20.0 m$^2$/g or more, or 25.0 m$^2$/g or more, and is preferably 35.0 m$^2$/g or less, or 32.0 m$^2$/g or less. The above upper and lower limits of the total pore area may be combined appropriately; for example, the total pore area may be 10.0 m$^2$/g or more and 35.0 m$^2$/g or less, 20.0 m$^2$/g or more and 35.0 m$^2$/g or less, or 25.0 m$^2$/g or more and 32.0 m$^2$/g or less.

The total pore volume of the shaped bodies of the present embodiment is preferably 0.35 mL/g or more, 0.36 mL/g or more, or 0.38 mL/g or more, and is preferably 0.70 mL/g or less, 0.60 mL/g or less, 0.50 mL/g or less, 0.48 mL/g or less, or 0.45 mL/g or less. The above upper and lower limits of the total pore volume may be combined appropriately; for example, the total pore volume may be 0.35 mL/g or more and 0.70 mL/g or less, 0.35 mL/g or more and 0.60 mL/g or less, 0.35 mL/g or more and 0.50 mL/g or less, 0.36 mL/g or more and 0.60 mL/g or less, 0.36 mL/g or more and 0.50 mL/g or less, 0.36 mL/g or more and 0.48 mL/g or less, or 0.38 mL/g or more and 0.45 mL/g or less.

The above total pore area and the above total pore volume may be combined appropriately, and the upper and lower limits of each may be an appropriate combination of the above. For example, the total pore area may be 10.0 m$^2$/g or more and 35.0 m$^2$/g or less, 20.0 m$^2$/g or more and 32.0 m$^2$/g or less, or 25.0 m$^2$/g or more and 32.0 m$^2$/g or less. The upper and lower limits of the total pore volume may be an appropriate combination of the above; for example, the total pore volume may be 0.35 mL/g or more and 0.50 mL/g or less, 0.36 mL/g or more and 0.48 mL/g or less, or 0.38 mL/g or more and 0.45 mL/g or less.

By virtue of having the total pore area or the total pore volume described above, the shaped bodies allow a dialysis effluent to penetrate easily therethrough and consequently tend to attain enhancements in potassium adsorption performance.

In the shaped bodies of the present embodiment, the pore size corresponding to 50% cumulative pore volume of the total pore volume (hereinafter, also written as the "pore size D50") is preferably 75 nm or more and 160 nm or less. The pore size D50 is more preferably 76 nm or more, 80 nm or more, or 100 nm or more, and is more preferably 155 nm or less, 120 nm or less, 118 nm or less, 116 nm or less, or 114 nm or less. The above upper and lower limits of the pore size D50 may be combined appropriately; for example, the pore size D50 may be 76 nm or more and 160 nm or less, 80 nm or more and 160 nm or less, 100 nm or more and 155 nm or less, 75 nm or more and 155 nm or less, 75 nm or more and 120 nm or less, 76 nm or more and 118 nm or less, 80 nm or more and 116 nm or less, or 100 nm or more and 114 nm or less.

The above ranges of pore size D50 more reliably provide high potassium adsorption performance.

In the shaped bodies of the present embodiment, the ratio of the cumulative pore volume of pores having a size of 5 nm or more and 10 nm or less (hereinafter, also written as the "5-10 nm pore volume ratio") to the total pore volume is preferably 0.1 vol% or more and 5.0 vol% or less. The 5-10 nm pore volume ratio is preferably 1.5 vol% or more, 2.2 vol% or more, or 3.1 vol% or more, and is preferably 4.9 vol% or less, 4.1 vol% or less, or 3.7 vol% or less. The above ranges of 5-10 nm pore volume ratio tend to ensure quick potassium adsorption. The above upper and lower limits of the 5-10 nm pore volume ratio may be combined appropriately; for example, the 5-10 nm pore volume ratio may be 1.5 vol% or more and 4.9 vol% or less, 2.2 vol% or more and 4.1 vol% or less, or 3.1 vol% or more and 3.7 vol% or less.

The pore areas, the pore volumes and the pore sizes of the shaped bodies may be measured by a mercury intrusion method in accordance with JIS Z 1655. Specifically, the measurement conditions may be as follows:

Mass of sample: 0.10 g
Mercury introduction pressure: 601.6 psia to 36,098.1 psia (4.1 MPa to 248.9 MPa)
Pore sizes measured: 5 nm to 300 nm
Cell used: Glass cell with an intrusion volume of 1.1 cc
Surface tension of mercury: 480 dyn
Contact angle of mercury: 130°
Pretreatment conditions: Degassing treatment in an air atmosphere at 110°C for at least 1 hour (1 hour or more and 3 hours or less).

The measurement may involve a general mercury porosimeter (for example, device name: AutoPore 9510, manufactured by Micromeritics).

The bulk density of the shaped bodies of the present embodiment is preferably 0.1 g/cm$^3$ or more and 0.65 g/cm$^3$ or less. When the bulk density is 0.1 g/cm$^3$ or more, the shaped bodies are easy to handle. When, on the other hand, the bulk density is 0.65 g/cm$^3$ or less, the flow of dialysis effluent is not excessively inhibited and the shaped bodies tend to be highly filled with the dialysis effluent. The bulk density of the shaped bodies of the present embodiment is preferably 0.1 g/cm$^3$ or more, 0.2 g/cm$^3$ or more, or 0.3 g/cm$^3$ or more, and is preferably 0.65 g/cm$^3$ or less, 0.60 g/cm$^3$ or less, or 0.58 g/cm$^3$ or less. The above upper and lower limits of the bulk density may be combined appropriately; for example, the bulk density may be 0.1 g/cm$^3$ or more and 0.65 g/cm$^3$ or less, 0.2 g/cm$^3$ or more and 0.60 g/cm$^3$ or less, or 0.3 g/cm$^3$ or more and 0.58 g/cm$^3$ or less.

The shaped bodies of the present embodiment may have a desired shape suited for the purpose. The shape of the shaped bodies of the present embodiment may be one or more selected from the group of cylindrical shapes, pellet-like shapes, bead-like shapes, roughly spherical shapes and ring-like shapes, and further may be at least any of cylindrical shapes and bead-like shapes. Any other shapes suited for the application may be adopted.

The shaped bodies of the present embodiment are suited for removing potassium from a dialysis effluent. For example, the potassium (K$^+$) removal ratio of the shaped bodies of the present embodiment in a potassium adsorption evaluation using a simulated dialysis fluid described below (hereinafter, the evaluation is also written as the "simulated adsorption evaluation") is preferably 20% or more, 30% or more, or 40% or more, and is preferably 100% or less, 99% or less, or 98% or less.

The simulated adsorption evaluation may be performed as follows. An aqueous solution containing KCl, NaCl, MgCl$_2$ and CaCl$_2$ and having the following composition is prepared as a simulated dialysis fluid.

K: 1.0 mEq/L
Na: 132.0 mEq/L
Mg: 0.5 mEq/L
Ca: 3.5 mEq/L

Next, 2.5 mL of the shaped bodies of the present embodiment are packed into a glass column with an inner diameter of 8.4 mmφ, and the simulated dialysis fluid is passed through the glass column at a flow rate of 100 mL/min. Every 100 mL of the simulated dialysis fluid discharged from the glass column is recovered from the start of discharge to collect a total of 500 mL of the simulated dialysis fluid, and each portion is diluted 10 times with pure water to give a measurement solution. The measurement solutions are each analyzed by ICP atomic emission spectrometry using a general ICP-AES device (for example, OPTIMA3000DV, manufactured by PERKIN-ELMER) to measure the concentrations of potassium, calcium and magnesium, and the potassium removal ratio is calculated from the following expressions (1) and (2):

$$\text{Potassium removal ratio [\%]} = (A1 + A2 + A3 + A4 + A5)/5 \times 100 \qquad (1)$$

In expression (1), A1 to A5 are the potassium removal ratios [%] of the respective measurement solutions.

$$\text{Potassium removal ratio [\%] of each measurement solution} = (C0 - Cn)/C0 \times 100$$

$$(2)$$

In equation (2), C0 is the potassium concentration [1.0 mEq/L] in the simulated dialysis fluid, and Cn is the potassium concentration [mEq/L] in each measurement solution.
Prior to the simulated adsorption evaluation, the shaped bodies of the present embodiment may be pretreated by being mixed with 1 L of an aqueous sodium chloride solution with a NaCl concentration of 10 mass% and then being washed with 1.5 L of pure water.
In the simulated adsorption evaluation, it is preferable that the magnesium removal ratio and the calcium removal ratio be lower than the potassium removal ratio; for example, the magnesium ($Mg^{2+}$) removal ratio may be 15% or less, 10% or less, or 6% or less, and the calcium ($Ca^{2+}$) removal ratio may be 15% or less, 10% or less, or 6% or less.
The lower limit of the magnesium removal ratio and that of the calcium removal ratio may be each, for example, 0% or more, 0.5% or more, or 1.0% or more.
The calcium removal ratio or the magnesium removal ratio may be calculated from expressions (1) and (2) while replacing potassium with calcium or magnesium.
The shaped bodies of the present embodiment release less aluminum when brought into contact with a solution, such as a dialysis effluent. For example, the amount of aluminum dissolved out from the shaped bodies of the present embodiment in an aluminum leaching evaluation below may be 0.01 ppm by mass or more and 0.50 ppm by mass or less, 0.01 ppm by mass or more and 0.20 ppm by mass or less, or 0.01 ppm by mass or more and 0.15 ppm by mass or less.
In the aluminum leaching evaluation, 1 mL of the shaped bodies of the present embodiment are mixed with 10 mL of pure water, and the mixture is shaken at 37°C for 6 hours at 1.0 ± 0.5 Hz. After the shaking, the supernatant is filtered through a membrane filter to give a measurement solution. The measurement solution is analyzed by ICP atomic emission spectrometry using ICP-AES (product name: OPTIMA3000DV, manufactured by PERKIN-ELMER) to determine the aluminum content, which is taken as the amount of aluminum leaching.
Prior to the aluminum leaching evaluation, the measurement sample may be pretreated by being mixed with 1 L of an aqueous sodium chloride solution with a NaCl concentration of 10 mass% and then being washed with 1.5 L of pure water.
The amount of aluminum leaching in the aluminum leaching evaluation is preferably small and, for example, may be 0 ppm by mass or more, or 0.01 ppm by mass or more.
The shaped bodies of the present embodiment are suited for removing potassium from a dialysis effluent and may be used in a potassium adsorption method that includes a step of contacting the shaped bodies with a solution containing potassium ions.
Furthermore, the shaped bodies of the present embodiment may be used as an adsorbent for selectively removing potassium from a solution containing at least two kinds of ions including alkali metal ions and alkaline earth metal ions. The shaped bodies of the present embodiment may be used as a potassium adsorbent, further as a potassium adsorbent from a solution containing at least any of sodium and magnesium, and potassium, and further as an adsorbent for removing potassium from at least any of biological fluids, blood products, blood and dialysis fluids.
The shaped bodies of the present embodiment may be packed into a column and the column containing the shaped bodies of the present embodiment may be used in the potassium adsorption method. Furthermore, the column may be used as part of a potassium adsorption system.

Next, a method for producing the shaped bodies of the present embodiment will be described.

The shaped bodies of the present embodiment may be produced by any method as long as the shaped bodies comprise an 8-membered ring zeolite and an inorganic binder source and have the configuration described hereinabove.

For example, a preferred production method includes a mixing step of mixing an 8-membered ring zeolite and an inorganic binder source to obtain a mixture, and a shaping step of shaping the mixture to obtain shaped bodies.

In the mixing step, the 8-membered ring zeolite and the inorganic binder source may be mixed by any process capable of mixing them uniformly. For example, the mixing process may employ one or more selected from the group of ribbon blenders, kneaders, Nauta mixers and Mix-Muller mixers.

The primary particle size and the particle size D50 of each of the 8-membered ring zeolite and the inorganic binder source subjected to the mixing step, and the primary particle size and the particle size D50 of the 8-membered ring zeolite and the inorganic binder contained in the shaped bodies of the present embodiment are not particularly limited as long as the shaped bodies described hereinabove can be obtained.

In the mixing step, the 8-membered ring zeolite and the inorganic binder are preferably mixed together so that the mass of the inorganic binder per 100 parts by weight (anhydrous equivalent mass) of the 8-membered ring zeolite will be 10 parts by mass or more, 15 parts by mass or more, or 20 parts by mass or more, and 40 parts by mass or less, 30 parts by mass or less, or 25 parts by mass or less.

The inorganic binder source may be a compound that functions as an inorganic binder by being fired into a compound containing silicon (Si) and oxygen (O), preferably a compound including silica ($SiO_2$), more preferably silica, or into a compound containing zirconium (Zr) and oxygen (O), preferably a compound including zirconia ($ZrO_2$), more preferably zirconia. The inorganic binder source is preferably one or more selected from the group of silica sol, colloidal silica, wet silica, dry silica, dry zirconia, wet zirconia, zirconia sol, zirconium hydroxide, zirconium carbonate, ammonium zirconium carbonate, zirconium oxynitrate, zirconium oxychloride, zirconium oxysulfate, zirconium acetate and zirconium oxyacetate, more preferably one or more selected from the group of colloidal silica, zirconium acetate, zirconium oxyacetate, wet zirconia, zirconia sol and zirconium hydroxide, and still more preferably at least any of colloidal silica and zirconia sol. Specifically, for example, the colloidal silica may be at least any of sodium ion-stabilized colloidal silica and ammonium ion-stabilized colloidal silica. For example, the inorganic binder source may be in the form of a solid, slurry, colloidal solution or aqueous solution containing the above compound.

In the mixing step, a shaping aid may be added as required. The shaping aid is a substance that improves shaping properties, and the use of the shaping aid facilitates controlling the pore structure of the shaped bodies. For example, the shaping aid may be one or more selected from the group of celluloses, alcohols, lignins, starches and guar gums. For easy handling, the shaping aid is preferably at least any of celluloses and alcohols. For example, the cellulose may be one or more selected from the group of crystalline celluloses, hydroxypropyl methylcellulose and sodium carboxymethylcellulose (CMC). For example, the alcohol may be at least any of polyvinyl alcohol and ethylene glycol.

The amount of the shaping aid per 100 parts by mass (anhydrous equivalent mass) of the zeolite is preferably 1 part by mass or more, or 2 parts by mass or more, and is preferably 5 parts by mass or less, or 4 parts by mass or less.

In the shaping step, the mixture obtained in the mixing step is shaped into a desired shape. The shaping process is not limited as long as the mixture can be formed into the desired shape. For example, cylindrical shaped bodies may be produced by extrusion, and bead-like shaped bodies may be produced by at least any of tumbling granulation and stirring granulation.

When producing shaped bodies with an average aspect ratio of greater than 1.55, the shaping step preferably involves a shaping process capable of producing a cylindrical shape, more preferably extrusion, to allow for easy control of the short and long axes of the shaped bodies. Specifically, for example, the shaping by extrusion may be performed using a general extruder (for example, product name: MG-55-1, manufactured by Dalton) to extrude the mixture obtained by the mixing step into a cylindrical shape with a diameter of 0.1 mm or more and 10.0 mm or less. The average aspect ratio tends to be increased as the diameter of the mixture being extruded is reduced.

When producing shaped bodies with an average aspect ratio of 1.55 or less, the shaping step preferably involves a shaping process capable of producing a roughly spherical or spherical shape, more preferably at least any of tumbling granulation and stirring granulation, to easily attain a small average aspect ratio. For example, the stirring granulation may be performed using a general stirring granulator (for example, product name: FM Mixer (FM5), manufactured by NIPPON COKE AND ENGINEERING CO., LTD.) to granulate the mixture obtained by the mixing step into bead-like shaped bodies.

Where necessary, the method for producing the shaped bodies of the present embodiment may include a firing step of heat-treating the shaped bodies. The firing step can increase the strength of the shaped bodies. The firing conditions are not limited as long as at least part of the inorganic binder source is melted and fuses with the 8-membered ring zeolite. For example, the firing may be performed at 500°C or above and 900°C or below for 2 hours or more and 4 hours or less. The firing atmosphere may be an oxidizing atmosphere, for example, an air atmosphere.

EXAMPLES

The present disclosure will be described in greater detail below based on Examples. However, the present disclosure is not limited to those Examples.

<Simulated adsorption evaluation>

The potassium adsorption performance of measurement samples was evaluated using a simulated dialysis fluid.

Specifically, an aqueous potassium solution containing KCl, NaCl, $MgCl_2$ and $CaCl_2$ and having the following composition was prepared as a simulated dialysis fluid.

    K: 1.0 mEq/L
    Na: 132.0 mEq/L
    Mg: 0.5 mEq/L
    Ca: 3.5 mEq/L

2.5 mL of the measurement sample was packed into a glass column with an inner diameter of 8.4 mmφ, and the simulated dialysis fluid was passed through the glass column at a flow rate of 100 mL/min.

Every 100 mL of the simulated dialysis fluid discharged from the glass column was recovered from the start of discharge to collect a total of 500 mL of the simulated dialysis fluid, and each portion was diluted 10 times with pure water to give a measurement solution.

The measurement solutions were each analyzed by ICP atomic emission spectrometry using an ICP-AES device (product name: OPTIMA3000DV, manufactured by PERKIN-ELMER) to measure the concentrations of potassium, calcium and magnesium, and the potassium removal ratio, the calcium removal ratio and the magnesium removal ratio were calculated from expressions (1) and (2) described hereinabove.

Prior to the simulated adsorption evaluation, the measurement sample was pretreated by being mixed with 1 L of an aqueous sodium chloride solution with a NaCl concentration of 10 mass% and then being washed with 1.5 L of pure water.

<Aluminum leaching evaluation>

The measurement samples were tested by the following method to evaluate the leaching of aluminum.

Specifically, 1 mL of the measurement sample was mixed with 10 mL of pure water, and the mixture was shaken at 37°C for 6 hours at 1.0 ± 0.5 Hz. After the shaking, the supernatant was filtered through a membrane filter to give a measurement solution. The measurement solution was analyzed by ICP atomic emission spectrometry using ICP-AES (product name: OPTIMA3000DV, manufactured by PERKIN-ELMER) to determine the aluminum content, which was taken as the amount of aluminum leaching.

Prior to the aluminum leaching evaluation, the measurement sample was pretreated by being mixed with 1 L of an aqueous sodium chloride solution with a NaCl concentration of 10 mass% and then being washed with 1.5 L of pure water.

<Measurement of pore sizes and pore volumes>

The pore volumes and the pore sizes of the measurement samples were measured by a mercury intrusion method in accordance with JIS Z 1655 using a mercury porosimeter (device name: AutoPore 9510, manufactured by Micromeritics). Specifically, the porosity and the pore sizes were measured under the following conditions:

    Weight of sample: 0.10 g
    Mercury introduction pressure: 601.6 psia to 36,098.1 psia (4.1 MPa to 248.9 MPa)
    Pore sizes measured: 5 nm to 300 nm
    Cell used: Glass cell with an intrusion volume of 1.1 cc
    Surface tension of mercury: 480 dyn
    Contact angle of mercury: 130°
    Pretreatment conditions: Degassing treatment in an air atmosphere at 110°C for at least 1 hour

<Measurement of particle size distribution>

The particle size distribution of the measurement samples was measured by the laser diffraction/scattering method. The measurement involved a general particle size distribution analyzer (device name: MT-3100II, manufactured by MicrotracBEL Corporation). The measurement conditions were as follows:

> Pretreatment: Drying in an air atmosphere at 110°C for 1 hour
> Measurement range: 0.7 to 1000 $\mu$m
> Transmissivity: Transmissive
> Shape: Aspheric
> Refractive index: 1.66
> Atmosphere: Air (dry)
> Solvent refractive index: 1

Example 1

100 Parts by mass (anhydrous equivalent mass: 300.0 g) of an FER-type zeolite (product name: HSZ (registered trademark)-720KOA, manufactured by TOSOH CORPORATION, $SiO_2/Al_2O_3$ ratio: 18, particle size D50: 8.7 $\mu$m), 20 parts by mass (anhydrous equivalent mass: 60.0 g) of sodium ion-stabilized colloidal silica (product name: ST-30, manufactured by Nissan Chemical Corporation, Si concentration in terms of $SiO_2$: 30 mass%, primary particle size: 12 nm) and 4 parts by mass (anhydrous equivalent mass: 12.0 g) of sodium-type carboxymethylcellulose (product name: Sunrose F-20LC, manufactured by Nippon Paper Industries Co., Ltd.) were stirred and mixed for 5 minutes using a stirring granulator (product name: FM Mixer (FM5), manufactured by NIPPON COKE AND ENGINEERING CO., LTD.). 105.4 g of pure water was added to the mixture and the resultant mixture was stirred for another 5 minutes. Subsequently, the mixture was dried in an air atmosphere at 100°C overnight and was classified using a Ro-Tap sieve shaker (product name: IIDA SHAVE SHAKER, manufactured by SIEVE FACTORY IIDA Co., Ltd.) under conditions where the number of impacts was 165 rpm, the rotational speed was 290 rpm and the processing time was 5 minutes. Shaped bodies that had passed through a 300 $\mu$m mesh sieve and had remained on a 180 $\mu$m mesh sieve were recovered. The shaped bodies recovered were fired in an air atmosphere at 600°C for 2 hours to give bead-like shaped bodies containing the FER-type zeolite (an 8-membered ring zeolite) and silica (a silica binder).

500.0 g of a 10 mass% aqueous NaCl solution was passed through the bead-like shaped bodies to perform ion exchange. Shaped bodies of this Example were thus obtained as Na cation-type shaped bodies.

Example 2

Shaped bodies of this Example were obtained in the same manner as in Example 1, except that shaped bodies that had passed through a 425 $\mu$m mesh sieve and had remained on a 300 $\mu$m mesh sieve were recovered.

Example 3

Shaped bodies of this Example were obtained in the same manner as in Example 1, except that the materials were mixed in a mixer (HIVIS MIX, manufactured by PRIMIX Corporation) for 60 minutes and the mixture was shaped with an extruder (product name: MG-55-1, manufactured by Dalton) into a 0.5 mm cylindrical shape, which was dried and crushed with a granulator (product name: FXB-3, manufactured by Fuji Paudal Co., Ltd.) at a hammer rotational speed of 15 Hz, and shaped bodies that had passed through a 425 $\mu$m mesh sieve and had remained on a 300 $\mu$m mesh sieve were recovered.

Example 4

Shaped bodies of this Example were obtained in the same manner as in Example 1, except that the sodium ion-stabilized colloidal silica was replaced by ammonium ion-stabilized colloidal silica (product name: ST-N-30G, manufactured by Nissan Chemical Corporation, Si concentration in terms of $SiO_2$: 30 mass%, primary particle size: 12.5 nm $\pm$ 2.5 nm).

Example 5

Shaped bodies of this Example were obtained in the same manner as in Example 1, except that the sodium ion-stabilized colloidal silica was replaced by ammonium ion-stabilized colloidal silica (product name: ST-N-30G, manufactured by Nissan Chemical Corporation, Si concentration in terms of $SiO_2$: 30 mass%, primary particle size: 12.5 nm $\pm$ 2.5 nm), and that shaped bodies that had passed through a 425 $\mu$m mesh sieve and had remained on a 300 $\mu$m mesh sieve were recovered.

Example 6

Shaped bodies of this Example were obtained in the same manner as in Example 1, except that the zeolite that was used was an FER-type zeolite (product name: HSZ (registered trademark)-720NHA, manufactured by TOSOH CORPORATION, $SiO_2/Al_2O_3$ ratio: 18, particle size D50: 10.9 $\mu$m) and the cation type thereof was changed to H-type by acid-treating the zeolite with 1 mol/L hydrochloric acid, and that the sodium ion-stabilized colloidal silica was replaced by ammonium ion-stabilized colloidal silica (product name: ST-N-30G, manufactured by Nissan Chemical Corporation, Si concentration in terms of $SiO_2$: 30 mass%, primary particle size: 12.5 nm $\pm$ 2.5 nm).

Example 7

Shaped bodies of this Example were obtained in the same manner as in Example 6, except that shaped bodies that had passed through a 425 $\mu$m mesh sieve and had remained on a 300 $\mu$m mesh sieve were recovered.

Example 8

100 Parts by mass (anhydrous equivalent mass: 300.0 g) of an FER-type zeolite (product name: HSZ (registered trademark)-720KOA, manufactured by TOSOH CORPORATION, $SiO_2/Al_2O_3$ ratio: 18, particle size D50: 8.7 $\mu$m), 20 parts by mass (anhydrous equivalent mass: 60.0 g) of a zirconia binder (product name: NanoUse ZR, manufactured by Nissan Chemical Corporation, Zr concentration in terms of $ZrO_2$: 40 mass%, primary particle size: 90 nm), and 4 parts by mass (anhydrous equivalent mass: 12.0 g) of sodium-type carboxymethylcellulose (product name: Sunrose F-20LC, manufactured by Nippon Paper Industries Co., Ltd.) were mixed together for 60 minutes using a mixer (HIVIS MIX, manufactured by PRIMIX Corporation). 252.5 g of pure water was added to the mixture and the resultant mixture was stirred for another 5 minutes. Subsequently, the mixture was shaped with an extruder (product name: MG-55-1, manufactured by Dalton) into a cylindrical shape 0.3 mm in diameter, which was dried in an air atmosphere at 100°C overnight and was crushed with a granulator (product name: FXB-3, manufactured by Fuji Paudal Co., Ltd.) at a hammer rotational speed of 15 Hz to give shaped bodies. The shaped bodies obtained were classified using a Ro-Tap sieve shaker (product name: IIDA SHAVE SHAKER, manufactured by SIEVE FACTORY IIDA Co., Ltd.) under conditions where the number of impacts was 165 rpm, the rotational speed was 290 rpm and the processing time was 5 minutes. Shaped bodies that had passed through a 710 $\mu$m mesh sieve and had remained on a 180 $\mu$m mesh sieve were recovered. The shaped bodies recovered were fired in an air atmosphere at 600°C for 2 hours to give cylindrical shaped bodies containing the FER-type zeolite (an 8-membered ring zeolite) and zirconia (a zirconia binder).

500.0 g of a 10 mass% aqueous NaCl solution was passed through the cylindrical shaped bodies to perform ion exchange. Shaped bodies of this Example were thus obtained as Na cation-type shaped bodies.

Example 9

Shaped bodies of this Example were obtained in the same manner as in Example 8, except that the zirconia binder was replaced by 20 parts by mass (anhydrous equivalent mass: 60.0 g) of sodium ion-stabilized colloidal silica (product name: SH-3, manufactured by FUSO CHEMICAL CO., LTD., Si concentration in terms of $SiO_2$: 34.4 mass%, primary particle size: 35.6 nm), that 174.0 g of pure water was added to the mixture, and that the shaped bodies recovered were fired in an air atmosphere at 800°C for 2 hours.

Example 10

Shaped bodies of this Example were obtained in the same manner as in Example 9, except that 10 parts by mass (anhydrous equivalent mass: 30.0 g) of the sodium ion-stabilized colloidal silica (product name: SH-3, manufactured by FUSO CHEMICAL CO., LTD., Si concentration in terms of $SiO_2$: 34.4 mass%, primary particle size: 35.6 nm) was added, that 230.1 g of pure water was added to the mixture, and that the mixture was shaped into a cylindrical shape with a diameter of 0.5 mm using the extruder.

Example 11

Shaped bodies of this Example were obtained in the same manner as in Example 9, except that 15 parts by mass (anhydrous equivalent mass: 45.0 g) of the sodium ion-stabilized colloidal silica (product name: SH-3, manufactured by FUSO CHEMICAL CO., LTD., Si concentration in terms of $SiO_2$: 34.4 mass%, primary particle size: 35.6 nm) was added, and that 63.6 g of pure water was added to the mixture.

Comparative Example 1

Shaped bodies of this Comparative Example were obtained in the same manner as in Example 1, except that an FAU-type zeolite (product name: HSZ (registered trademark)-320NAA, manufactured by TOSOH CORPORATION, $SiO_2/Al_2O_3$ ratio: 5.7, particle size D50: 6.3 $\mu$m) was used as the zeolite.

Comparative Example 2

Shaped bodies of this Comparative Example were obtained in the same manner as in Example 1, except that shaped bodies that had passed through a 1.2 mm mesh sieve and had remained on a 1.0 mm mesh sieve were recovered.

Comparative Example 3

Commercially available FER-type zeolite shaped bodies (product name: HSZ (registered trademark)-720KOD1 C, 1.5 mm$\varphi$ cylindrical pellets, binder: clay, $SiO_2/Al_2O_3$ ratio: 18, particle size D50: (zeolite) 8.7 $\mu$m, (binder) 12 $\mu$m) were crushed in a mortar. Cylindrical pellet-like shaped bodies that had passed through a 425 $\mu$m mesh sieve and had remained on a 300 $\mu$m mesh sieve were recovered.
500.0 g of a 10 mass% aqueous NaCl solution was passed through the cylindrical pellet-like shaped bodies to perform ion exchange. Shaped bodies of this Comparative Example were thus obtained as Na cation-type shaped bodies.

Comparative Example 4

100 Parts by mass of an MOR-type zeolite (product name: HSZ (registered trademark)-620HOA, manufactured by TOSOH CORPORATION, $SiO_2/Al_2O_3$ ratio: 18, particle size D50: 10.1 $\mu$m), 20 parts by mass (anhydrous equivalent mass: 60.0 g) of sodium ion-stabilized colloidal silica (product name: SH-3, manufactured by FUSO CHEMICAL CO., LTD., Si concentration in terms of $SiO_2$ : 34.4 mass%, primary particle size: 35.6 nm) and 4 parts by mass (anhydrous equivalent mass: 12.0 g) of sodium-type carboxymethylcellulose (product name: Sunrose F-20LC, manufactured by Nippon Paper Industries Co., Ltd.) were mixed together for 60 minutes using a mixer (HIVIS MIX, manufactured by PRIMIX Corporation). 95.1 g of pure water was added to the mixture and the resultant mixture was stirred for another 5 minutes. Subsequently, the mixture was shaped with an extruder (product name: MG-55-1, manufactured by Dalton) into a 1.0 mm cylindrical shape, which was dried in an air atmosphere at 100°C overnight and was crushed with a granulator (product name: FXB-3, manufactured by Fuji Paudal Co., Ltd.) at a hammer rotational speed of 15 Hz to give shaped bodies. The shaped bodies obtained were classified using a Ro-Tap sieve shaker (product name: IIDA SHAVE SHAKER, manufactured by SIEVE FACTORY IIDA Co., Ltd.) under conditions where the number of impacts was 165 rpm, the rotational speed was 290 rpm and the processing time was 5 minutes. Shaped bodies that had passed through a 1.2 mm mesh sieve and had remained on a 1.0 mm mesh sieve were recovered. The shaped bodies recovered were fired in an air atmosphere at 600°C for 2 hours to give cylindrical shaped bodies containing the MOR-type zeolite (an 8-membered ring zeolite) and silica (a silica binder).
500.0 g of a 10 mass% aqueous NaCl solution was passed through the cylindrical shaped bodies to perform ion exchange. Shaped bodies of this Example were thus obtained as Na cation-type shaped bodies.
The evaluation results of Examples and Comparative Examples are described in the following tables.

[Table 1]

| Nos. | Structures | Binders | Amount of binder [parts] | Bulk density [g/cm³] | Aspect ratio | Short axis [µm] | Particle size D50 [µm] | Total pore volume [mL/g] | Total pore area [m²/g] | Pore size D50 [nm] | 5-10 nm pore volume ratio [%] | 10-100 nm pore volume ratio [%] |
|---|---|---|---|---|---|---|---|---|---|---|---|---|
| Ex. 1 | FER | Na ion-stabilized silica binder | 20 | 0.52 | 1.35 | 239 | 264 | 0.48 | 26.8 | 117 | 2.2 | 33.9 |
| Ex. 2 | FER | Na ion-stabilized silica binder | 20 | 0.58 | 1.39 | 327 | 417 | 0.47 | 30.6 | 111 | 4.1 | 37.1 |
| Ex. 3 | FER | Na ion-stabilized silica binder | 20 | 0.49 | 1.48 | 385 | 462 | 0.46 | 25.0 | 117 | 1.5 | 33.7 |
| Ex. 4 | FER | NH3 ion-stabilized silica binder | 20 | 0.53 | 1.31 | 223 | 259 | 0.43 | 28.8 | 84 | 2.2 | 65.0 |
| Ex. 5 | FER | NH3 ion-stabilized silica binder | 20 | 0.50 | 1.36 | 358 | 377 | 0.44 | 31.7 | 83 | 3.1 | 64.9 |
| Ex. 6 | FER | NH3 ion-stabilized silica binder | 20 | 0.55 | 1.38 | 215 | 251 | 0.36 | 30.5 | 76 | 4.9 | 63.1 |
| Ex. 7 | FER | NH3 ion-stabilized silica binder | 20 | 0.53 | 1.48 | 308 | 405 | 0.37 | 28.9 | 79 | 3.7 | 60.8 |
| Ex. 8 | FER | Zirconia binder | 20 | 0.48 | 2.15 | 379 | 505 | 0.59 | 24.9 | 155 | 0.0 | 27.2 |
| Ex. 9 | FER | Na ion-stabilized silica binder (high purity) | 20 | 0.43 | 2.39 | 368 | 570 | 0.59 | 33.1 | 118 | 0.0 | 39.8 |
| Ex. 10 | FER | Na ion-stabilized silica binder (high purity) | 10 | 0.47 | 1.60 | 508 | 615 | 0.64 | 28.9 | 139 | 0.0 | 31.1 |
| Ex. 11 | FER | Na ion-stabilized silica binder (high purity) | 15 | 0.50 | 2.52 | 620 | 699 | 0.59 | 36.3 | 108 | 0.0 | 44.7 |

| Nos. | Structures | Binders | Amount of binder [parts] | Bulk density [g/cm$^3$] | Aspect ratio | Short axis [$\mu$m] | Particle size D50 [$\mu$m] | Total pore volume [mL/g] | Total pore area [m$^2$/g] | Pore size D50 [nm] | 5-10 nm pore volume ratio [%] | 10-100 nm pore volume ratio [%] |
|---|---|---|---|---|---|---|---|---|---|---|---|---|
| Comp. Ex. 1 | FAU | Na ion-stabilized silica binder | 20 | 0.67 | 1.35 | 334 | 401 | 0.30 | 8.8 | 152 | 0.0 | 13.2 |
| Comp. Ex. 2 | FER | Na ion-stabilized silica binder | 20 | 0.56 | 1.55 | 970 | >1000 upper detection limit | 0.45 | 36.9 | 104 | 7.4 | 39.0 |
| Comp. Ex. 3 | FER | Clay | 20 | 0.58 | 2.23 | 276 | 418 | 0.43 | 34.5 | 65 | 0.0 | 95.8 |
| Comp. Ex. 4 | MOR | Na ion-stabilized silica binder (high purity) | 20 | 0.66 | 1.87 | 1130 | >1000 upper detection limit | 0.08 | 11.4 | 28 | 0.0 | 79.5 |

[Table 2]

| No. | K+ removal ratio [%] | Ca2+ removal ratio [%] | Mg2+ removal ratio [%] | Amount of aluminum leaching [ppm] |
|---|---|---|---|---|
| Ex. 1 | 57.0 | 0.7 | 0.5 | 0.07 |
| Ex. 2 | 44.0 | 1.8 | 0 | 0.08 |
| Ex. 3 | 41.0 | 0 | 0.5 | 0.17 |
| Ex. 4 | 64.0 | 2.4 | 0.6 | 0.19 |
| Ex. 5 | 48.0 | 0 | 0.5 | 0.20 |
| Ex. 6 | 49.0 | 0 | 0 | 0.02 |
| Ex. 7 | 33.0 | 0.7 | 0 | 0.05 |
| Ex. 8 | 32.7 | 2.2 | 0.5 | 0.003 |
| Ex. 9 | 25.6 | 0.5 | 0.2 | 0.001 |
| Ex. 10 | 26.4 | 0.3 | 0 | 0 |
| Ex. 11 | 24.1 | 3.1 | 1.4 | 0.006 |
| Comp. Ex. 1 | 21.9 | 10.5 | 23 | 0.12 |
| Comp. Ex. 2 | 13.9 | 0.5 | 0.3 | 0.20 |
| Comp. Ex. 3 | 53.5 | -39.3 | 2.5 | 0.45 |
| Comp. Ex. 4 | 6.2 | 0 | 0 | 0.00 |

INDUSTRIAL APPLICABILITY

The shaped bodies of the present disclosure have a pore size distribution and a particle size that are useful for adsorbing and removing potassium ions, and thus may be usefully used to adsorb potassium contained in a solution, preferably to adsorb potassium from a potassium ion-containing effluent discharged during dialysis.

The entire contents of the description, the claims and the abstract of Japanese Patent Application No. 2022-176351, filed on November 2, 2022, and the entire contents of the description, the claims and the abstract of Japanese Patent Application No. 2023-132534, filed on August 16, 2023, are hereby incorporated by reference as the disclosure of the specification of the present disclosure.

**Claims**

1. Shaped bodies comprising a zeolite having an oxygen 8-membered ring structure, and at least any of a silica binder and a zirconia binder, the shaped bodies being such that the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies satisfy (1) or (2) below:

   (1) the average aspect ratio is 1.55 or less, and
   the particle size D50 is 200 $\mu$m or more and 600 $\mu$m or less;
   (2) the average aspect ratio is greater than 1.55.

2. The shaped bodies according to claim 1, wherein the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of greater than 1.55.

3. The shaped bodies according to claim 1, wherein the shaped bodies comprise a zeolite having an oxygen 8-membered ring structure, and at least any of a silica binder and a zirconia binder, the ratio of the cumulative pore volume of pores having a size of 10 nm or more and 100 nm or less to the cumulative pore volume of pores having a size of 5 nm or more and 300 nm or less is 25 vol% or more and 70 vol% or less, and the shaped bodies have an average aspect ratio of 1.55 or less and a particle size D50 of 200 $\mu$m or more and 600 $\mu$m or less.

4. The shaped bodies according to any one of claims 1 to 3, wherein the cumulative pore area of pores having a size of 5 nm or more and 300 nm or less is 10.0 $m^2$/g or more and 35.0 $m^2$/g or less.

5. The shaped bodies according to any one of claims 1 to 4, wherein the cumulative pore volume of pores having a size of 5 nm or more and 300 nm or less is 0.35 mL/g or more and 0.70 mL/g or less.

6. The shaped bodies according to any one of claims 1 to 5, wherein the pore size corresponding to 50 vol% cumulative pore volume of the cumulative pore volume of pores having a size of 5 nm or more and 300 nm or less is 75 nm or more and 160 nm or less.

7. The shaped bodies according to any one of claims 1 to 6, wherein the ratio of the cumulative pore volume of pores having a size of 5 nm or more and 10 nm or less to the cumulative pore volume of pores having a size of 5 nm or more and 300 nm or less is 0.1 vol% or more and 5.0 vol% or less.

8. The shaped bodies according to any one of claims 1 to 7, wherein the zeolite has one or more framework structures selected from the group of FER structures, HEU structures, YFI structures, MAZ structures, CHA structures and MOR structures.

9. The shaped bodies according to any one of claims 1 to 8, wherein the zeolite has a silica to alumina molar ratio (a $SiO_2$/$Al_2O_3$ ratio) of 3 or more and 100 or less.

10. A potassium adsorption method comprising a step of contacting the shaped bodies described in any one of claims 1 to 9 and a solution containing potassium ions with each other.

11. A column comprising the shaped bodies described in any one of claims 1 to 9.

12. A potassium adsorption system comprising the column described in claim 11.

## INTERNATIONAL SEARCH REPORT

<table>
<tr><td>International application No.</td></tr>
<tr><td>**PCT/JP2023/038862**</td></tr>
</table>

**A. CLASSIFICATION OF SUBJECT MATTER**

*C01B 39/44*(2006.01)i; *B01D 15/00*(2006.01)i; *A61M 1/16*(2006.01)i; *A61M 1/36*(2006.01)i; *B01J 20/18*(2006.01)i; *B01J 20/28*(2006.01)i

FI:     B01J20/18 E; C01B39/44; B01D15/00 P; B01J20/18 C; A61M1/16 190; B01J20/28 Z; A61M1/36 165

According to International Patent Classification (IPC) or to both national classification and IPC

**B. FIELDS SEARCHED**

Minimum documentation searched (classification system followed by classification symbols)

B01D15/00; A61M1/16; A61M1/36;C01B33/20-C01B33/46; C01B37/00-C01B39/54; B01J20/00-B01J20/28; B01J20/30-B01J20/34

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2010-29741 A (NATIONAL INSTITUTE OF ADVANCED INDUSTRIAL SCIENCE AND TECHNOLOGY) 12 February 2010 (2010-02-12) claims, paragraphs [0010], [0016], [0019] | 1-2, 8-12 |
| Y | | 10-12 |
| A | | 3-7 |
| Y | US 2015/0297815 A1 (TRIOMED AB) 22 October 2015 (2015-10-22) paragraph [0052] | 10-12 |
| A | | 1-9 |
| A | JP 58-133804 A (UNION CARBIDE CORP.) 09 August 1983 (1983-08-09) | 1-12 |
| A | JP 2012-66241 A (MITSUBISHI CHEMICAL CORP.) 05 April 2012 (2012-04-05) | 1-12 |

☑ Further documents are listed in the continuation of Box C.      ☑ See patent family annex.

| | |
|---|---|
| *    Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **04 December 2023** | **09 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2023/038862** |

**C.    DOCUMENTS CONSIDERED TO BE RELEVANT**

| Category* | Citation of document, with indication, where appropriate, of the relevant passages | Relevant to claim No. |
|---|---|---|
| A | JP 2021-510623 A (ALBEMARLE CORP.) 30 April 2021 (2021-04-30) | 1-12 |
| A | US 6235960 B1 (INSTITUT FRANCAIS DU PETROLE) 22 May 2001 (2001-05-22) | 1-12 |
| A | WO 99/13981 A1 (TOYOTA JIDOSHA KABUSHIKI KAISHA) 25 March 1999 (1999-03-25) | 1-12 |
| A | JP 2022-161381 A (HITACHI ZOSEN CORP.) 21 October 2022 (2022-10-21) | 1-12 |
| A | JP 57-72653 A (UNION CARBIDE CORP.) 07 May 1982 (1982-05-07) | 1-12 |

Form PCT/ISA/210 (second sheet) (January 2015)

**INTERNATIONAL SEARCH REPORT**
Information on patent family members

International application No.

**PCT/JP2023/038862**

| Patent document cited in search report | | | Publication date (day/month/year) | Patent family member(s) | | | Publication date (day/month/year) |
|---|---|---|---|---|---|---|---|
| JP | 2010-29741 | A | 12 February 2010 | (Family: none) | | | |
| US | 2015/0297815 | A1 | 22 October 2015 | WO | 2014/081367 | A1 | |
| JP | 58-133804 | A | 09 August 1983 | EP | 87223 | A1 | |
| JP | 2012-66241 | A | 05 April 2012 | (Family: none) | | | |
| JP | 2021-510623 | A | 30 April 2021 | WO | 2019/140249 | A1 | |
| | | | | CN | 111565841 | A | |
| US | 6235960 | B1 | 22 May 2001 | EP | 962251 | A1 | |
| WO | 99/13981 | A1 | 25 March 1999 | US | 6559086 | B1 | |
| JP | 2022-161381 | A | 21 October 2022 | (Family: none) | | | |
| JP | 57-72653 | A | 07 May 1982 | EP | 46971 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150297815
- JP 2022176351 A
- JP 2023132534 A